(19) Europäisches Patentamt
European Patent Office
Office européen des brevets

(11) EP 0 741 709 B1

(12) EUROPEAN PATENT SPECIFICATION

(45) Date of publication and mention of the grant of the patent:
**25.07.2001 Bulletin 2001/30**

(21) Application number: **95910135.3**

(22) Date of filing: **24.01.1995**

(51) Int Cl.[7]: **C07D 233/88**, A61K 31/415, A61K 31/335, A61K 31/495, A61K 31/535, C07D 403/12, C07D 405/12, C07D 413/12

(86) International application number:
**PCT/US95/01150**

(87) International publication number:
**WO 95/19968 (27.07.1995 Gazette 1995/32)**

(54) **AROMATIC 2-AMINO-IMIDAZOLE DERIVATIVES AS ALPHA-2A ADRENOCEPTOR AGONISTS**

AROMATISCHE 2-AMINO-IMIDAZOLDERIVATE ALS ALPHA 2A-ADRENOCEPTOR AGONISTEN

DERIVES AROMATIQUES 2-AMINO-IMIDAZOLE UTILISES COMME AGONISTES DE L'ADRENORECEPTEUR ALPHA-2A

(84) Designated Contracting States:
**DE ES FR GB IT**

(30) Priority: **24.01.1994 US 186406**
**24.01.1994 US 185653**

(43) Date of publication of application:
**13.11.1996 Bulletin 1996/46**

(73) Proprietor: **Allergan Sales, Inc.**
**Irvine, CA 92612 (US)**

(72) Inventors:
- **MUNK, Stephen, A.**
  **Irvine, CA 92714 (US)**
- **GARST, Michael, E.**
  **Newport Beach, CA 92660 (US)**
- **HARCOURT, Dale, A.**
  **San Clemente, CA 92672 (US)**
- **GLUCHOWSKI, Charles**
  **Wayne, NJ 07470 (US)**

(74) Representative: **Hutchins, Michael Richard et al**
**FRY HEATH & SPENCE**
**The Old College**
**53 High Street**
**Horley Surrey RH6 7BN (GB)**

(56) References cited:
**WO-A-92/04345**

- **CHEMICAL ABSTRACTS, vol. 70, no. 11, 17 March 1969, Columbus, Ohio, US; abstract no. 47457q, N. GRUENFELD 'Imidazoles.' page 339 ;column 2 ; cited in the application & GB,A,1 131 191 (GEIGY, J.R. A.-G.) 23 October 1968**
- **JOURNAL OF MEDICINAL CHEMISTRY, vol.35, no.4, 21 February 1992, WASHINGTON US pages 750 - 755 Y. AMEMIYA ET AL. 'Synthesis and alpha-Adrenergic Activities of 2- and 4-Substituted Imidazoline and Imidazole Analogues'**
- **CHEMICAL AND PHARMACEUTICAL BULLETIN, vol.33, no.10, October 1985, TOKYO JP pages 4409 - 4421 M. MATSUO ET AL. 'New Aryliminoimidazolidines. I. Synthesis and Antihypertensive Properties of 2-(2-Phenoxyphenylimino)imidazolidines and Related Compounds'**
- **MORRISON R.T.; BOYD R.N.: "Organic Chemistry", 3rd Edition, 1975, p. 596, Allyn and Bacon, Boston, US**
- **SYKES P.: "A Guidebook to Mechanism in Organic Chemistry", 5th Edition, 1981, pp. 345-378, Longman, London, GB**
- **WELLS P.R.: "LInear Free Energy Relationships", 1968, p. 14, Academic Press, London, GB**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 741 709 B1

## Description

Field of the Invention

The present invention relates to substituted phenyl-2-amino-imidazoles. More particularly, the invention relates to such compounds which are useful in binding to and activating the $\alpha_{2A}$ adrenoreceptor which are therapeutically useful in the treatment of peripheral pain, elevated intraocular pressure and hypertension, as adjuncts in anesthesia, and as sedatives.

Background Of The Invention

A few phenyl-2-amino-imidazole derivatives are known in the pharmaceutical arts:

GB-A-1,131,191 discloses a class of phenyl-2-amino- or phenylalkyl-2-amino compounds as having a number of pharmacological properties. Jen, et al. in J. Med. Chem., 18 (1), 90-99 (1975) made and tested the compounds below for antihypertensive and gastric antisecretory activity. These compounds were among a wider group of cyclic and acyclic amidines studied, however only the compounds shown have features in common with the present invention.

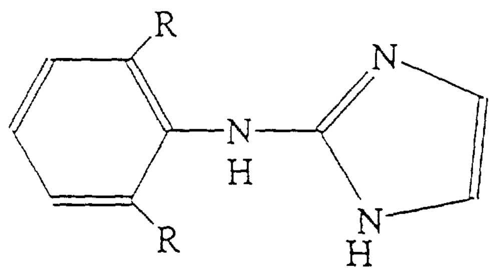

Both R groups are the same and are H, Cl, or $CH_3$

U.S. Patent number 3,459,763 (to Gruenfeld) discloses a variety of substituted imidazole compounds, the main classes of compounds disclosed are regioisomers of the general structures: phenyl-2-amino-imidazoles and 1-N-phenyl-2-amino-imidazoles. Both classes of compounds appear to be produced by acid-induced cyclization from the same intermediates. Testing of the p-fluoro analog which is outside the scope of the present invention showed that it possessed modest hypotensive activity. It is not clear from the specification whether both regioisomers are formed as a mixture and are separated by recrystallization or whether some intermediate structures favor formation of one regioisomer over the other.

The background of the division of adrenoceptors into differing categories and subtypes can be briefly described as follows. Historically, adrenoceptors were first divided in $\alpha$ and $\beta$ types by Ahlquist in 1948. This division was based on pharmacological characteristics. Later, $\beta$-adrenoceptors were subdivided into $\beta_1$ and $\beta_2$ subtypes, again based on a pharmacological definition by comparison of the relative potencies of 12 agonists. The $\alpha$-adrenoceptors were also subdivided into $\alpha_1$ and $\alpha_2$ subtypes, initially based on a presumed localization of $\alpha_1$ receptors postsynaptically and $\alpha_2$ presynaptically. Now, however, this physiologic division is no longer used and it is generally accepted that the most useful way to subdivide the $\alpha$-adrenoceptors is based on pharmacology using affinities for the antagonists yohimbine and prazosin. At $\alpha_1$ receptors, prazosin is more potent than yohimbine, whereas at $\alpha_2$ receptors, yohimbine is more potent than prazosin. Bylund, et al. first suggested in 1981 that there possibly existed subtypes of the $\alpha_2$-adrenoceptors on the basis of radioligand binding studies. This initial work was done with various tissues taken from various species. While receptor heterogeneity among species is considered to be important, the term 'subtype' is usually reserved by pharmacologists for heterogeneity which can be demonstrated within the same species and ideally within a single tissue. Bylund and coworkers have later demonstrated that some regions of the human and rat brain contain two populations of $\alpha_2$-adrenoceptor sites which differ in their affinity for prazosin by 30- to 40-fold. This finding supports the division of the $\alpha_2$ receptor into A and B subtypes.

Some examples of well known alpha$_2$ ($\alpha_2$) adrenergic receptor selective compounds known in the art are:

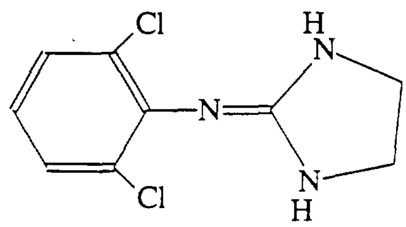

Clonidine

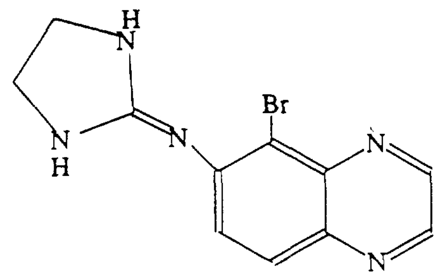

Brimonidine

Clonidine is clinically useful as a hypotensive agent, and has been studied as a nasal decongestant and as an ocular hypotensive agent and as an anesthetic adjunct. The mechanism of action of clonidine has been described as a centrally acting $\alpha_2$ adrenergic partial agonist. Brimonidine (UK 14,304) is a newer, more lipophilic $\alpha_2$ adrenergic agent that is also being studied as an antihypertensive, ocular hypotensive, and in other $\alpha_2$ agonist responsive conditions.

Still other examples of known $\alpha_2$ selective agonists that have been reported to have $\alpha_{2A}$ selectivity are:

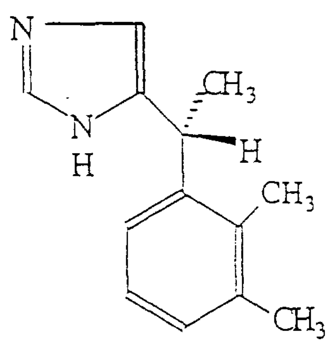

Dexmedetomidine

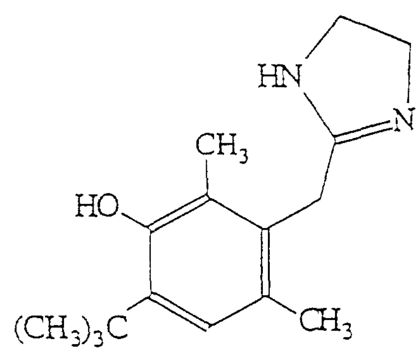

Oxymetazoline

Reports of studies of alpha 2A adrenoceptor activity for the compounds shown above are: Takano Y., et al., Eur. J. Pharmacol. 219 (3) pp. 465-68 (1992); Angel I., et al., J. Pharmacol. Exp. Ther. 254 (3) pp. 877-82 (1990), and Hirose, H., et al., J. Lab. Clin. Med. 121(1) pp.11-12 (1993). The studiesexamined $\alpha$2A receptors in relation to pain transmission and hyperglycemia.

A selective agonist as the term is used in this invention indicates a compound that binds to, and activates, a specific receptor subtype in preference to other receptors of related but different subtype(s). For example, a compound that binds to and activates the $\alpha_{2A}$ subtype receptor in preference to the $\alpha_{2B}$ or $\alpha_{2C}$ subtype receptors is an $\alpha_{2A}$ selective agonist. Activation means that the receptor is induced to initiate a biochemical event that is controlled or operated by that particular receptor. Activation can further be thought of in terms of inducing the receptor to express its function.

The identification of subtypes of the alpha 2 receptors has progressed faster than the pharmacological and physiological characterization of these subtypes. However, the $\alpha_{2A}$ receptor has been identified in the ciliary body of the eye , and so is postulated to have a controlling mechanism in glaucoma (see Jin, Y. et al., J. Ocul. Pharmacol., 10 (1) pp. 359-69 (1994)). It has. also been studied in pain perception, or alternatively, pain alleviation (see Millan, M. J., Eur. J. Pharmacol., 215(2-3) pp. 355-6 (1992)).

Summary of the Invention

In the present invention, it has been found that compounds of the formula I, as defined in the claims appended here to, are selective agonists for $\alpha_{2A}$ adrenoceptors.

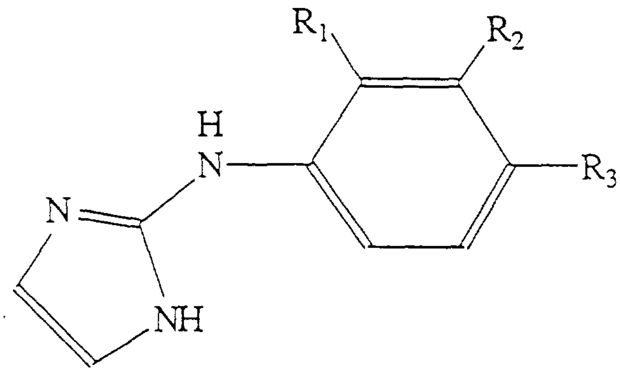

I

The $\alpha_{2A}$ selectivity of these compounds is greatly enhanced by the substituent present on the benzene or fused aromatic ring at the position occupied by group $R_1$ in formula I. Without wishing to be held to a specific theory on this effect, it is believed that the ortho substituent induces a "twist" (variation in the angle made by the intersection of the planes of the phenyl and imidazole rings) of the imidazole ring relative to the benzene ring. Alpha$_{2A}$ selectivity is further enhanced when the substituents at $R_1$, $R_2$, or $R_3$ are capable of electron donation to the aromatic ring. In formula I, $R_1$ is alkyl or alkenyl of 1 to 4 carbons, cyclopropyl, $-OR_4$, $-SR_4$, $-NR_4R_4$ or $-Si(R_4)_3$ wherein $R_4$ represents alkyl or alkenyl of 1 to 4 carbon atoms or is hydrogen, $R_2$ and $R_3$ are selected from the group consisting of $R_1$ and hydrogen; or $R_1$ and $R_2$ taken together form a fused ring with the phenyl moiety and are selected from the group consisting of $-(CH_2)_4-$, $-NH(CH_2)_2NH-$, $-O(CH_2)_2O-$, $-NH(CH_2)_2O-$, $-S(CH_2)_2S-$, $-NH(CH_2)S-$, and $-O(CH_2)_2S-$ and $R_3$ is selected from the group consisting of $R_1$ and hydrogen; or $R_2$ and $R_3$ taken together form a fused ring with the phenyl moiety and are selected from the group consisting of $-(CH_2)_4-$, $-NH(CH_2)_2NH-$, $-O(CH_2)_2O-$, $-NH(CH_2)_2O-$, $-S(CH_2)_2S-$, $-NH(CH_2)S-$, and $-O(CH_2)_2S-$, -CH=CH-CH=CH-, -N=CH-CH=N- and -N=CH-N=CH-, or pharmaceutically acceptable salts thereof.

For the foregoing unsymmetrical atom strings representing $R_1/R_2$ or $R_2/R_3$, the attachment atoms at either end can bind in either orientation to the ring. For example with -CH=N=CH=N-, the first carbon atom can bond to the benzene ring at $R_2$ and the ending nitrogen atom can bond at $R_3$, or the reverse is also possible (last nitrogen to $R_2$ and first carbon to $R_3$).

Further these compounds are useful in treating conditions which are linked to adrenergic control through the $\alpha_{2A}$ receptor. Examples of conditions which can be treated are: peripheral pain, reduction or maintenance of intraocular pressure or the symptoms of glaucoma in at least one eye, and cardiovascular hypertension. Other therapeutic uses are for sedation, in treating hyperglycemia, and in augmentation of the effects of anesthetics.

Thus, the invention also relates to a compound of the formula I for use in a method of selectively stimulating adrenergic $\alpha_{2A}$ receptors in human or non-human mammals comprising administering to a mammal requiring $\alpha_{2A}$ adrenoceptor stimulation, a therapeutically effective amount of a compound or mixture of compounds of formula I or pharmaceutically acceptable salts thereof. The compounds of the present invention are useful to provide one or more desired therapeutic effects in a mammal.

Pharmaceutically acceptable salts of the compounds of formula I are also within the scope of the present invention. Pharmaceutically acceptable acid addition salts of the compounds of the invention are those formed from acids which form non-toxic addition salts containing pharmaceutically acceptable anions, such as the hydrochloride, hydrobromide, hydroiodide, sulfate, bisulfate, phosphate or acid phosphate, acetate, maleate, fumarate, oxalate, lactate, tartrate, citrate, gluconate, saccharate, or p-toluenesulfonate salts. A pharmaceutically acceptable salt may be any salt which retains the activity of the parent compound and does not impart any deleterious or untoward effect on the subject to which it is administered and in the context in which it is administered.

Organic amine salts may be made with amines, particularly ammonium salts such as mono-, di- and trialkyl amines or ethanol amines. Salts may also be formed with caffeine, tromethamine, and similar molecules. Where there is a nitrogen sufficiently basic as to be capable of forming acid addition salts such may be formed with any inorganic or organic acids or alkylating agent such as methyl iodide. Any of a number of simple organic acids such as mono-, di-, or tri-acid may also be used. A pharmaceutically acceptable salt may be prepared for any compound of the invention having a functionality capable of forming such a salt, e.g., an acid salt of an amine functionality.

General Embodiments

Definitions

The compounds of the present invention all contain the (2-imidazolyl)amino structure as follows:

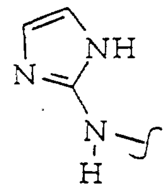

this group is attached by the exocyclic nitrogen to an aromatic ring. Other adrenergic compounds are also known in the art which do not have the imidazole ring. Compounds such as the following:

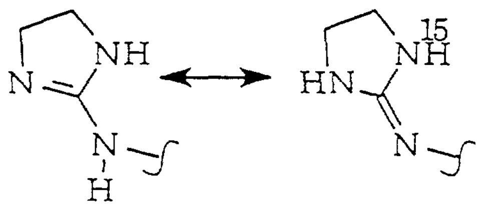

exist as tautomers wherein the double bond can shift from one nitrogen to another. The chemical nomenclature of these compounds is: 2-amino-imidazolines and 2-iminoimidazolidines (from left to right). No tautomeric form of these compounds places a double bond at the 4-5 position of the imidazole ring.

The term "alkyl" as used here refers to and includes normal and branch chained alkyl groups. The term "lower alkyl", unless specifically stated otherwise, includes normal alkyl of 1 to 6 carbons, branch chained alkyl of 3 to 6 carbons. Similarly, the terms "alkenyl" and "alkynyl" include normal and branch chained groups having 2 to 6 carbons when the chains are normal, and 3 to 6 carbons when the chains are branched.

Detailed Description of the Invention

The compounds of this invention, with reference to Formula I are those where $R_1$ is not hydrogen, and it is preferred that $R_1$ and/or $R_2$, and /or $R_3$ are substituents which are capable of electron donation to the aromatic ring. Groups which are capable of electron donation to the ring include rings fused to the aryl ring at the positions occupied by $R_1$ and $R_2$ or $R_2$ and $R_3$.

An empirical measure of the electron-donating ability of single and multi-atom substituents on aromatic, particularly benzene rings, are Hammett values (taken from the sigma value in the Hammett equation :

$$\log\left(\frac{K}{K^{\circ}}\right) = \rho\sigma.$$

Another measurement for individaul atoms is electronegativity. This is a measure of the electron donating or withdrawing capacity of individual atoms, however, these values are not available for multi-atoms substituents and measure the "pull" on electrons an atom makes in a covalent bond. The Hammett sigma values measure the influence of the substituent effect on reactivity of substrates and have been measured for substituents meta and para to the substrate group on benzene rings. Tables of these measurements can be found in organic and physical organic textbooks, for instance, Vogel's Handbook of Organic Chemistry. In many cases these values are measured by a comparison of the acid dissociation constants of the appropriately substituted aromatic carboxylic acid with the unsubstituted aromatic carboxylic acid. For substituents at the ortho position, owing to a neighbor effect from close proximity of the substituent to the reaction center on the ring, that $\sigma_{ortho}$ values cannot be reliably measured in every case and so are not published. However, for the purposes of defining this invention $\sigma_{meta}$ values give a measure of electron donation to the ring of a

substituent at the ortho position, neighbor effects notwithstanding. The σ value for hydrogen is zero, as would be expected since these measurements are a comparison of a compound with substituents against the unsubstituted, i. e. "hydrogen substituted" compound. Applicants know of no better empirical measure of electron donation to an aromatic ring for defining the scope of the present invention, which includes multi-atom substituents and ring fusion. Therefore, the use of $\sigma_{meta}$ values as measured by means well known in the art are relied upon for a measure of the electron donation by substituents to both the phenyl and fused aromatic ring systems.

Descriptions of experimental methods for measuring σ values not available in published lists can be found in Advanced Organic Chemistry, J. March, McGraw Hill Book Company, pp. 251-253 (1977). For a review of the Hammett equation see Jaffé, Chem. Rev. **53**, 191 (1953). Tables of rho (p) values for the equations can be found in Wells, Chem. Rev. **63**, 171-218 (1963) and Bekkum ,Verkade and Wepster, Recl. Trav. Chim. Pavs Bas **78**, 821-827 (1959).

Preferred compounds of the invention are those compounds with an ortho substituent having a $\sigma_{meta}$ of 0.4 or less. Still more preferred are those compounds that have an electron donating group with $\sigma_{meta}$ values of about 0.15 or less (including those substituents that have negative σ values) at $R_1$. Even more preferred are those compounds with electron releasing groups located at $R_2$ and / or $R_3$, as well as at $R_1$. Most preferred are those compounds which have a saturated heterocyclic ring fused to the benzene ring at the positions occupied by $R_2$ and $R_3$ in addition to an electron donating group with a $\sigma_{meta}$ value of about 0.15 or less (including those substituents that have negative σ values) at $R_1$.

Scheme I shows a general synthetic pathway to obtain the 2-aminoimidazole compounds of the present invention. R1, R2, and R3 are as defined in Formula I above. Briefly, an optionally substituted aniline (aminobenzene) is treated with a strong base such as potassium hydride in tetrahydrofuran which then is allowed to react with phenylcyanate. The resulting N-cyano-aniline is refluxed with aminoacetaldehyde diethyl acetal in ethanol with methanesulfonic acid or another protic acid to produce the N-(2,2Diethoxyethyl)-N"-(anilino)guanidine. The diethoxy moiety is hydrolyzed in 20% HCl and then cyclized to form the imidazole ring by treatment with an aqueous base such as NaOH to give the N-(2-imidazolyl)aminobenzene compounds with varying substituents on the benzene ring. By starting with a ring system which bears an amine group as an alternative to aminobenzenes , such as the 6-aminoquinoxaline ring, other compounds, in this instance the 6-(N-cyano)aminoquinoxaline, can be produced.

SCHEME I

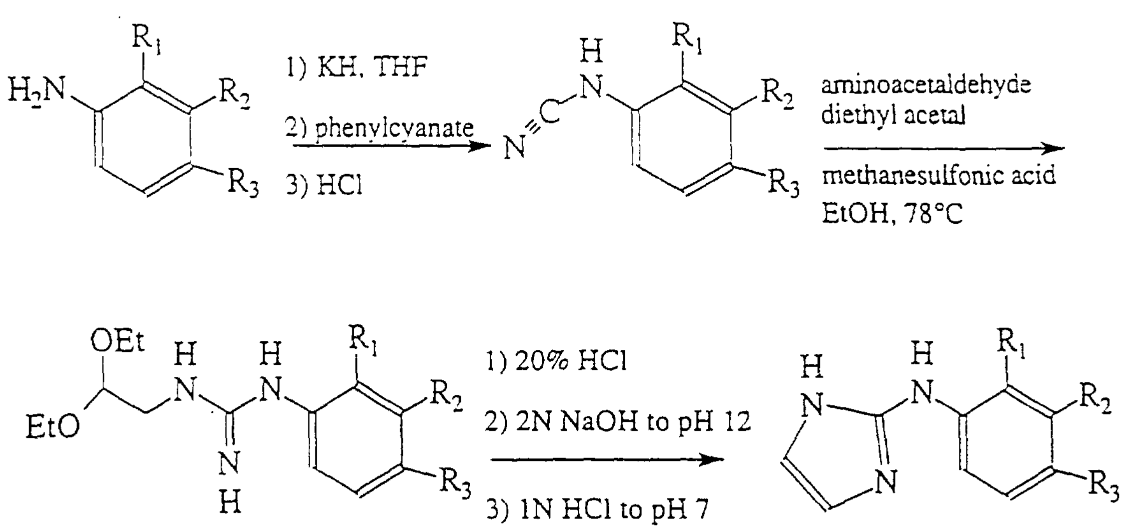


The invention is further illustrated by the following non-limiting examples which are illustrative of a specific mode of practicing the invention and are not intended as limiting the scope of the appended claims.

Example 1: 2-N-(phenyl)amino-1H-imidazole

This example illustrates the general synthetic methodology although the product of the reaction sequence is not within the scope of formula I.

**A.**

N-cyanoaniline

Aniline (5.00g, 53.7 mmol) was dissolved in anhydrous ether (25 mL) and pentane (25 mL) and cooled to 0° C. The cyanogen bromide 10.00 g, 105 mmol) was added to the cooled aniline solution and stirred overnight at room temperature. The mixture was concentrated to give a slurry product. Ether was added to the slurry and the resulting precipitate was filtered off. The filtrate was concentrated to give pure N-cyanoaniline (4.6 g, 72% yield).

**B.**

N-(2,2-diethoxyethyl)-N'-(phenyl)guanidine

The cyanamide (4.6 g, 39 mmol) was dissolved in anhydrous ethanol (100 mL). To this solution was added aminoacetaldehyde diethylacetal (Aldrich, 15.5 g 117 mmol) and methanesulfonic acid (Aldrich, 4.14 g, 39 mmol). The mixture was heated at reflux until starting material was no longer observed via thin layer chromatography (48 h). The crude reaction mixture was concentrated in vacuo and purified by flash chromatography on silica with 5% methanol saturated with ammonia/chloroform to give N-(2,2-diethoxyethyl)-N'-(phenyl)guanidine (6.5 g, 66%) as a thick oil which was carried on to the next step without further purification.

**C.**

2-N-(phenyl)amino-1H-imidazole

The acetal of previous step (1.3 g, 5.17 mmol) was dissolved in a solution of concentrated hydrochloric acid (Mallinkcrodt, 37%, 10 mL) and water (10 mL) at O° C for 1 h. The solution was made basic (pH = 14) by the addition of 6N sodium hydroxide and stirred for 15 min. The resulting solution was extracted in methylene chloride, concentrated and purified by flash chromatography on silica with 5% methanol saturated with ammonia/chloroform to give 2-N-(phenyl)amino-1H-imidazole (35 mg, 4.5%).

**Example 2: 5-Methyl-6-N-(2-imidazolyl)aminoquinoxaline (6)**

**A.**

5-Methyl-6-(N-cyano)aminoquinoxaline (4)

To a slurry of potassium hydride (3.70g, 92.27 mmol) in anhydrous tetrahydrofuran (20 mL) at -78°C, 6-amino-5-methylquinoxaline (3) (6.37 g, 40.12 mmol)was added in THF (60 mL) via cannula. This mixture was stirred at 0°C for 1 h. After gas evolution had subsided, phenyl cyanate (5.25g, 44.13 mmol) was added via syringe. This mixture was stirred for an additional 4 h at 0°C. Anhydrous diethyl ether (30 mL) was added to the stirring solution and the resulting precipitate was filtered off, dissolved in H2O, hot filtered, neutralized to pH 6 with HCl (6N) and the resulting precipitate was filtered off. HCl was also added dropwise to the mother liquor until a precipitate formed. The precipitate was filtered off, dissolved in $H_2O$, hot filtered, neutralized to pH 6 with HCl and this precipitate was collected and combined with the other solids. The combined solids were slurried in diethyl ether for 1 h and filtered off. These solids were dried in vacuo to give pure 4 (4.98g) in 68% yield.

$^1H$ NMR(DMSO): 2.57 (s, 3h), 7.65 (d, J=9.3 Hz, 1H), 7.98 (d, J=3.9Hz, 1H), 8.83 (s, 1H), 8.90 (s, 1H), 9.90-10.10 (brs, 1H).

$^{13}C$ NMR(DMSO): 10.40, 112.32, 119.80, 120.36, 128.19, 137.10, 138.97, 141.54, 143.59, 145.18

IR(KBr): 3220, 2239, 1618, 1500 $cm^{-1}$

MS: Exact mass calculated for $C_{10}H_8N_4$ ($M^+$) 184.0748, found 184.0762.

**B.**

N-(2,2Diethoxyethyl)-N"-[6-(5-methylquinoxalinyl)]guanidine (5)

To a slurry of 4 (1.43g, 7.76 mmol) in anhydrous ethanol (120 mL) in a 2-neck round-bottom flask equipped with reflux condenser and Drierite™ drying column was added aminoacetaldehyde diethylacetal (4.13g, 31.03 mmol) and methanesulfonic acid (Aldrich, 0.75g, 7.76 mmol). The mixture was heated at reflux until starting material was no

longer observed by thin layer chromatography (48 h). The crude reaction mixture was concentrated in vacuo and purified by flash chromatography on silica with 10% methanol saturated with ammonia/chloroform to give 5 as a pale foam which was carried on to the next reaction without further purification.

$^1H$ NMR($CDCl_3$): 1.21 (t, J=7.0Hz, 6H), 2.55 (s, 3H), 3.43 (d, J=5.5Hz, 2H), 3.52-3.64 (m, 2H), 3.65-3.80 (m, 2H), 4.61 (t, J=5.5Hz, 1H), 4.90-5.20 (brs, 3H), 7.37 (d, J=8.8Hz, 1H), 7.72 (d, J=8.8Hz, 1H), 8.55 (d, J=1.8Hz, 1H), 8.65 (d, J=1.8Hz, 1H).

MS: Exact mass calculated for $C_{14}H_{18}N_5O_2(M^{+\cdot}-C_2H_5^+)$ 288.1460, found 288.1458.

**C.**

5-Methyl-6-N-(2-imidazolyl)aminoquinoxaline (6)

The acetal (5) was dissolved in a solution of concentrated HCl (Mallinkrodt 37%, 15 mL) and water (15mL) at O° C. Occasionally a colorless precipitate would form soon after the addition of HCl and would be removed by filtration. The resulting solution was stirred for 1h while warming to ambient temperature. The solution was made basic (pH=14) by addition of 6N sodium hydroxide and stirred for 15 min. before bringing the pH back to neutral by the addition of 1N HCl. The yellow precipitate was filtered off and filtered through silica using 5% ammonia saturated $MeOH/CHCl_3$ to give pure product. The mother liquor was lyophilized and product remaining in the mother liquor residue was extracted into methanol and the NaCl was filtered off. This crude product was likewise filtered through silica and both pure portions were combined to give pure 6 (1.46g, 6.49 mmol) in 79% yield through two steps.

Elemental analysis: Calcd for C12H11N5:

| | |
|---|---|
| theoretical | H 4.92 C 63.98 N 31.09 |
| found | H 4.83 C 63.74 N 30.95 |

$^1H$ NMR(DMSO): 2.62 (s, 3h), 6.70-6.90 (brs, 2H), 7.81 (d, J=9.3 Hz, 1H), 8.30 (s, 1H), 8.44 (d, J=9.3 Hz, 1H), 8.66 (d, J=1.8 Hz, 1H), 8.80 (d, J=1.8Hz, 1H), 11.0 (brs, 1H).

**Example 3: 5-Bromo-6-N-(2-imidazolyl)aminoquinoxaline** (Comparative Example)

**A.**

5-Bromo-6-(N-cyano)aminoquinoxaline, sodium salt

To a solution of 6-amino-5-bromoquinoxaline (6.61 g, 29.4 mmol) in anhydrous tetrahydrofuran (150 mL) at ambient temperature was added sodium hydride (Aldrich 60% suspension in oil, 1.77 g, 73.5 mmol) in portions. As gas evolution began to diminish the mixture was heated to reflux for 90 min with stirring. The reaction was cooled to ambient temperature and phenyl cyanate was added via syringe. This mixture was refluxed for an additional 2 h before concentrating to 50% of its original volume. The resulting precipitate was collected by filtration and rinsed with diethyl ether and dried in vacuo to give 5-bromo-6-(N-cyano) aminoquinoxaline, sodium salt (6.60 g), a yellow-green solid in 83% yield.

$^1H$ NMR(DMSO): 7.57 (d, J= 9.1 Hz, 1H), 7.64 (d, J= 9.1 Hz, 1H), 8.36 (d, J=2.0 Hz, 1H), 8.63 (d, J=2.0 Hz, 1H).

MS: Exact mass calculated for $C_9H_5BrN_4$ ($M^+$) 247.9697, found 247.9700.

**B.**

N-(2,2-Diethoxyethyl)-N'-[6-(5-bromoquinoxalinyl)] guanidine

To a solution of 5-bromo-6-(N-cyano)aminoquinoxaline, sodium salt (0.26 g, 0.94 mmol) in anhydrous ethanol (25 mL) in a 2-neck round-bottom flask equipped with a reflux condenser and Drierite™ drying column was added aminoacetaldehyde diethylacetal (Aldrich, 0.19 g, 1.39 mmol) followed by methanesulfonic acid (Aldrich, 0.11 g, 1.13 mmol). The mixture was heated at reflux until starting material was no longer observed via thin layer chromatography (24 h). The crude reaction mixture was concentrated in vacuo, taken up in ethyl acetate, washed with saturated sodium bicarbonate, water and brine. The organic portions were filtered and re concentrated to a brown oil which was purified by flash chromatography on silica with 40% tetrahydrofuran/ethyl acetate to give N-(2,2-diethoxyethyl)-N'-[6-(5-bromoquin oxalinyl)]guanidine (0.27 g, 0.70 mmol) in 74% yield. Small amounts of impurities were co-eluted with the product but did not interfere with subsequent reactions.

$^1H$ NMR($CDCL_3$): 1.23 (t, J= 7.0 Hz, 6H), 3.46 (d, J=5.0 Hz, 2H), 3.55-3.70 (m, 2H), 3.71-3.83 (m, 2H) 4.70 (t, J= 5.0 Hz, 1H), 4.90 (brs, 1H), 5.25 (brs, 2H), 7.46 (d, J=9.0 Hz, 1H), 7.80 (d, J= 9.0 Hz, 1H), 8.60 (d, J= 1.9 Hz, 1H), 8.76 (d, J= 1.9 Hz, 1H)

MS: Exact mass calculated for $C_{13}H_{15}BrN_5O_2$ ($M^+$-$C_2H_5^+$) 352.0409, found 352.0401.

**C.**

5-Bromo-6-N-(2-imidazolyl)-aminoquinoxaline

The compound of example 6 (3.10 g, 8.14 mmol) was dissolved in a solution of concentrated hydrochloric acid (37%, 15 mL) and water (15 mL) at 0° C. The resulting solution was stirred for 2 h while warming to ambient temperature. The solution was then made basic (pH = 13) by the addition of 2N sodium hydroxide and stirred for 10 min. before bringing the pH back to neutral by the addition of 1N hydrochloric acid. The yellow-green crude product was filtered off and the mother liquor was lyophilized. Crude product remaining in the mother liquor residue was extracted into methanol and the sodium chloride was removed by filtration. The crude product portions were combined and adhered to silica with methanol prior to flash chromatography on silica with 10% methanol/chloroform to give pure 5-bromo-6-N-(2-imidazolyl)aminoquinoxaline (1.86 g, 6.41 mmol) in 79% yield

$^1H$ NMR(DMSO): 6.65-6.70 (brs, 2H), 7.99 (d, J= 9.3 Hz, 1H), 8.62 (s, 1H), 8.73 (d, J=9.3 Hz, 1H), 8.74 (d, J=1.9 Hz, 1H), 8.89 (d, J=1.9 Hz, 1H), 11.29 (brs, 1H).

Elemental analysis: Calcd for $C_{11}H_8BrN_5$:

| | |
|---|---|
| theoretical | H 2.78 C 45.54 N 24.14 |
| found | H 2.82 C 45.73 N 23.90 |

**Scheme II**

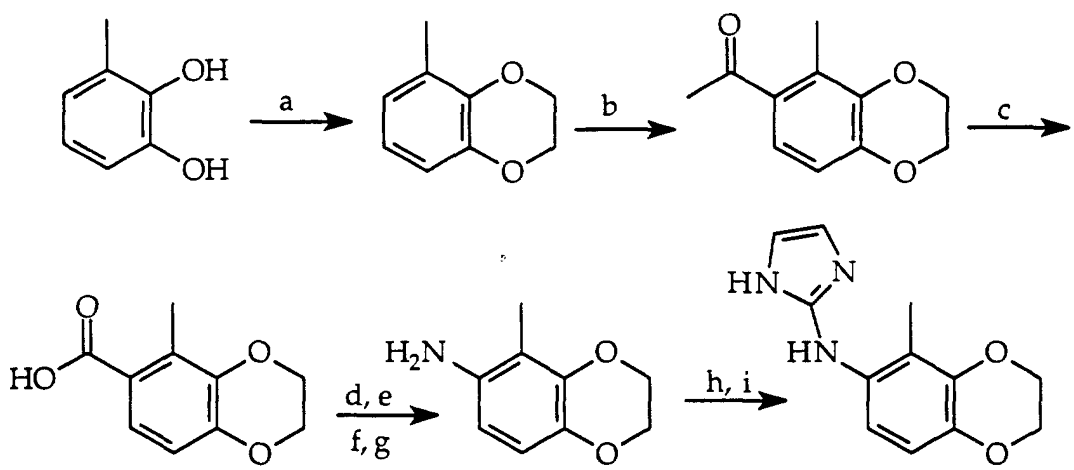


Reagents: a) Br-$CH_2$-$CH_2$-Br, NaI (5 mol%), acetone; b) $CH_3C(O)OC(O)CH_3$, PPA; c) NaClO; d) $EtO_2CCl$, diisopropylethylamine; e) $NaN_3$; f) benzyl alcohol, Δ; g) $H_2$, Pd-C; h) N,N-2,2-(diethoxy)-ethylcarbodiimide, $CH_3SO_3H$; i) HCl, $H_2O$

Example 4 gives experimental details of the synthesis shown in Scheme II which provides 5-methyl-6-N-(2-imidazolyl)-amino-1,4-benzodioxane.

**Example 4: 5-Methyl-6-N-(2-imidazolyl)-amino-1,4-benzodioxane**

**A.**

5-Methyl-1,4-benzodioxane

A mixture of 3-methylcatechol (12.4 g, 100 mmol, Aldrich), 1,2-dibromoethane (9.5 mL, 110 mmol, Aldrich)

and potassium carbonate (34.6 g, 0.25 mol) in acetone was warmed at reflux with mechanical stirring in a Morton Flask under argon overnight. Starting material was present so sodium iodide (0.75 g, 5.0 mmol) was added. The mixture was warmed for an additional 48 hrs. at which point the black slurry was poured into water and extracted with ether 3 times; the ether layer was washed one time with 5% sodium thiosulfate, dried over $MgSO_4$, passed through a pad 1 inch deep by 1/2 inch wide of silica (well washed with ether), concentrated and distilled (Kugelrohr, 80° C, 0.05 mm Hg) to afford 10.5 g gold-colored oil, which showed slight impurities by NMR. Flash chromatography (400 mL $SiO_2$ in a 2 inch by eight inch column, eluted with 5% ethyl acetate in hexane) afforded 9.0 g of pure product.

**B.**

5-Methyl-6-acetyl-1,4-benzodioxane

A mixture of the benzodioxane (A) (60 mmol, 9.0 g) and acetic anhydride (60 mmol, 6.13 g, Aldrich) was treated with polyphosphoric acid (76.4 g) at 55 - 62° C in a heating bath with mechanical mixing under nitrogen for 90 minutes. The reaction was cooled and quenched by the addition of 100 mL of water. The reaction mixture was poured into water and extracted three times with ether. The combined ether fraction were washed two times with saturated $NaHCO_3$ solution, one time with saturated NaCl solution, dried over $MgSO_4$, and concentrated to afford an oily semi-solid. Chromatography ($SiO_2$ in a 2 inch by 6 inch column, eluted with 10% ethyl acetate in hexane) provided 4.0 g crystalline solid, 1.5 g of a pure oil and a 2 g mixture which was also an oil. 1H NMR suggested that the crystalline fraction was the desired product. X-ray crystallography confirmed this assignment.

**C.**

5-Methyl-6-carboxy-1,4-benzodioxane

Fresh sodium hypochlorite was prepared by treating $Ca(ClO)_2$ (80 mmol, 11.43 g) in 50 mL of water with $Na_2CO_3$ (76 mmol, 8.05 g) and NaOH (24 mmol, 0.96 g) in 25 mL water. To this solution which resulted after warming with mixing and subsequent filtration was added solid acetophenone (example A, preceding) (20 mmol, 3.84 g). This mixture was warmed overnight at 60 ° C in a heating bath. The aqueous material was added to a separatory funnel and washed two times with dichloromethane. The material was then transferred to an Erlenmeyer Flask and treated dropwise with concentrated HCl until the solution reached a pH of 3. The solid was collected by filtration and washed well with water. The NMR was consistent with the proposed product.

**D.**

5-Methyl-6-N-(2-imidazolyl)-amino-1,4-benzodioxane

A mixture of diisopropyl-ethylamine (25 mmol, 4.35 mL), and the acid from the preceding example (20 mmol, 3.95 g) in acetone was treated dropwise with ethyl chloroformate (21 mmol, 2.01 mL) in an ice bath over 10 minutes. After 30 minutes, at 0° C, a solution of $NaN_3$ (40 mmol, 2.6 g, ACS Reagent) in water was added dropwise over 10 minutes. After 45 minutes at 0° C, the reaction mixture was poured into ice water and washed 3 times with dichloromethane. This solution was dried over $Na_2SO_4$ and concentrated to afford an oil that was warmed in toluene and benzyl alcohol at reflux for 2 hours. The reaction mixture was again concentrated to an oil to yield the 6-benzyl urethane of 5-methylbenzodioxane. The amine was liberated by treatment of the urethane with 200 mg of 10% Pd on carbon in THF and hydrogen gas.

Using the free amine (D), the named product was made analogously to Example 3 using the procedures described in Parts A, B, and C above.

$^1H$ NMR(DMSO): 2.00 (s, 3H), 4.12-4.23 (dd, 4H, J=7.14 Hz, 25.8 Hz), 6.55 (d, 1H, J=11.48 Hz), 6.6 (s, 2H), 7.14 (d, 1H, J=8.79 Hz), 7.45 (s, 1H), 10.4 - 10.6 (s, 1H).

$^{13}C$ NMR(DMSO): 9.73, 63.52, 64.36, 110.83, 113.69, 114.56, 134.82, 137.63, 141.24, 146.48

MS: HRMS exact mass calcd. for $C_{12}H_{13}N_3O_2$ ($M^+$) 231.1007 found: 231.1008

Elemental analysis: Calcd for $C_{12}H_{13}N_3O_2$:

| | |
|---|---|
| theoretical | H 5.67 C 62.32 N 18.17 |
| found | H 5.57 C 62.11N 18.08 |

## Example 5: Experimental Assays: Binding Affinities and Receptor Activation

Receptor Binding Assays

A.

Tissue preparation: Membrane suspensions were prepared from human cerebral cortex (HCC, for $\alpha_1$ receptors) obtained from the UCI Organ and Tissue Bank. Briefly, tissues (1g) were homogenized in 25 mL of ice-cold 5 mM tris, pH 7.4 with a Polytron homogenizer for 30 sec at setting #7, and centrifuged for 10-12 minutes at 300 x g a 4°C. The supernatant was filtered through 2 layers of gauze and diluted 1:2 with 50 mM Tris-HCl buffer, pH 7.4, then centrifuged at 49,000 x g for 20 minutes. The pellet fraction was washed 3 times (resuspended in Tris-HCl buffer and centrifuged for 20 minutes at 49,000 x g). The pellet was then stored at -80°C until the binding assay.

Cell preparation: Chinese hamster ovary (CHO) cells expressing the human $\alpha_{2A}$ and human $\alpha_{2C}$ (CHO-C10 and CHO-C4 respectively) receptors and CHO cells (CHO-RNG) expressing the rat $\alpha_{2B}$ adrenoceptor were grown to near confluence in Dulbecco's modified Eagle's medium supplemented with 10% fetal bovine serum using standard cell culture methods. Cells were harvested by scraping and placed into cold buffer of the following composition: 50 mM Tris-HCl, 5 mM EDTA, pH 7.4). Cells were then homogenized with a Polytron homogenizer for 2 X 10 sec at setting #7, and centrifuged for 20 minutes at 49,000 x g. The pellet fraction was washed (resuspended in Tris-HCl, pH 8 buffer and centrifuged for 15-20 minutes at 49,000 x g) 2 times and stored at -100°C until binding assay.

Binding Studies: The radioligands [$^3$H]rauwolscine (specific activity 80 Ci/mmol) and [$^3$H]prazosin (specific activity 76 Ci/mmol) were obtained from New England Nuclear, Boston, MA. Frozen membrane pellet was resuspended in 25 mM glycine/glycine, pH 7.5 and incubated with radioligand under the following conditions: CHO-C10, CHO-RNG, CHO-C4-[$^3$H]rauwolscine, 22°C, 30 minutes; RKC-[$^3$H]rauwolscine, 0°C, 120 minutes; and, HCC-[$^3$H]prazosin, 22°C, 30 minutes in a final volume of 500 ul. At the end of the incubation period, the samples were filtered through glass filters (Whatman GF/B) in a 96 well cell harvester and rapidly washed four times with 4 mL of iced-cold 50 mM Tris-HCl buffer. The filters were then oven dried and transferred to scintillation vials containing 10 mL of Beckman's Ready Protein® scintillation cocktail for counting. Specific binding defined by 10 uM phentolamine for competition studies were as follows: 2.4 nM [$^3$H]brimonidine-RbICB 62%; 2.4 nM [$^3$H]rauwolscine-RbICB 75%; 2 nM [$^3$H]rauwolscine-RbKc 88%; 0.3 nM [$^3$H] rauwolscine-CHO-C10 99%; 0.4 nM [$^3$H]rauwolscine-CHO-RNG 99%, 0.3 nM [$^3$H]prazosin 87%; and 1 nM [$^3$H]rauwolscine-CHO-C4 90%. Protein concentrations were determined with a protein assay kit from Bio Rad. Binding isotherms, equilibrium dissociation and affinity constants were analyzed and determined by the non-linear least squares curve fitting programs EBDA (BioSoft) or AccuFit Competition/Saturation by Beckman.

B.

Cell preparation: Chinese hamster ovary (CHO) cells expressing the human $\alpha_{2A}$ (CHO-C10) and the rat $\alpha_{2B}$ (CHO-RNG) human $\alpha_{2A}$adrenoceptors were grown to near confluence in Dulbecco's modified Eagle's medium supplemented with 10% fetal bovine serum using standard cell culture methods. Cells were harvested by scraping and placed into cold buffer of the following composition: 50 mM Tris-HCl, 5 mM EDTA, pH 7.4). Cells were then homogenized with a Polytron homogenizer for 2 X 10 sec at setting #7, and centrifuged for 20 minutes at 49,000 x g. The pellet fraction was washed (resuspended in Tris-HCl, pH 8 buffer and centrifuged for 15-20 minutes at 49,000 x g) 2 times and stored at -100°C until binding assay.

Binding studies: Determination of Ki

The radioligands [$^3$H)rauwolscine (specific activity 80 Ci/mmol) and [$^3$H]prazosin (specific activity 76 Ci/mmol) were obtained from New England Nuclear, Boston, MA. Frozen membrane pellet was resuspended in 25 mM glycine/glycine, pH 7.4 and incubated with radioligand under the following conditions: CHO-C10, CHO-RNG, CHO-C4-[$^3$H] rauwolscine, 22°C, 30 min.; and, HCC-[$^3$H]prazosin, 22°C, 30 minutes in a final volume of 500 ul. At the end of the incubation period, the samples were filtered through glass fiber filters (Whatman GF/B) in a 96 well cell harvester and rapidly washed four times with 4 mL of iced-cold 50 mM Tris-CHl buffer. The filters were then oven dried and transferred to scintillation vials containing 5 mL of Beckman's Ready Protein® scintillation cocktail for counting. Specific binding defined by 10 uM phentolamine for competition studies were as follows: 0.3 nM[$^3$H] rauwolscine-CHO-C10 99%; 0.4 nM [$^3$H]rauwolscine-CHO-RNG 99%, and 0.3 nM [$^3$H] prazosin-HCC 87%. Protein concentrations were determined with a protein assay kit from Bio Rad. Binding isotherms, equilibrium dissociation and affinity constants were analyzed and determined by the non-linear least squares curve fitting programs AccuFit Competition/Saturation by Beckman.

Functional Experiments

*Vas Deferens:* The prostatic ends of the vas deferens (2-3 cm) were removed from albino rabbits and mounted between platinum electrodes in 9 mL organ baths containing Krebs-Hensleit solution of the following composition (mM): NaCl 119, KCl 4.7, $MgSO_4$ 1.5, $KH_2PO_4$ 1.2. $CaCl_2$ 2.5, $NaHCO_3$ 25 and glucose 11. This solution was maintained at 35° C and bubbled with 95% $O_2$ and 5% $CO_2$. The tissue was equilibrated at 0.5 g tension for 30 minutes. The vas deferens strips were then field stimulated at 0.1 Hz, 2 msec, 90 mA using a square wave stimulator (World Precision Instruments A310 Accupulser / A385 Stimulus Isolater), or a Grass S48 stimulator at 0.1 Hz, 2 msec, 70 volts. After 30 minutes of electrical stimulation, cumulative concentration-response curves in 0.25 log units were obtained with a 4 minute contact time for each concentration. Each tissue was used to evaluate only one drug. Tissue contractions produced by the field stimulation were measured isometrically using Grass FT-.03 force-displacement transducers and recorded on a Grass Model 7D physiograph. The reduction in electrically-evoked peak height by the drugs was measured and expressed as a percentage of the pre-drug peak height. The $IC_{50}$ was determined as the concentration which produced a 50% reduction in peak height.

Representative compounds of the present invention, and comparative examples, were tested according to the procedures given above. Results of these tests are tabulated below. The receptor binding studies and $K_i$ are measures of the affinity of a compound for a particular receptor. The vas deferens assay is a measure of the degree of activation of the $\alpha_{2A}$ affected when a compound binds to to that receptor. Rabbit vas deferens has been found to have predominantly the $\alpha_{2A}$ receptor subtype.

## TABLE 1

| Structure | $K_i$ (nM) | | | | $EC_{50}$(nM) |
|---|---|---|---|---|---|
| | $\alpha_1$ human brain | $\alpha_{2A}$ CHO-C10 | $\alpha_{2B}$ CHO-RNG | $\alpha_{2C}$ CHO-C4 | rabbit vas deferens |
| 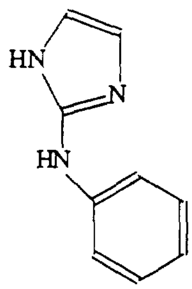 | 67,520 | 394 | 1,888 | 3,236 | 56,200 |
| 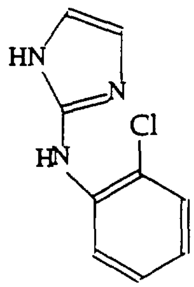 | 23,210 | 287 | 1,942 | 2,250 | |
| 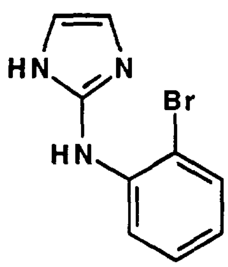 | 9,975 | 214 | 1,451 | 2,392 | |
| 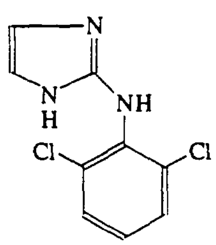 | 5,937 | 95 | 587 | 1,118 | 86.3 |
| 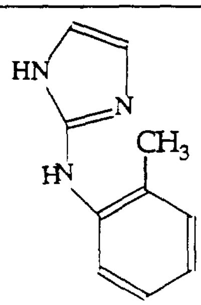 | 10,620 | 73 | 561 | 577 | |

Table 1 (con't.)

| ***Structure*** | *α1* *human brain* | *α2A* *CHO-C10* | *α2B* *CHO-RNG* | *α2C* *CHO-C4* | ***EC50*** *rabbit vas deferens* |
|---|---|---|---|---|---|
| 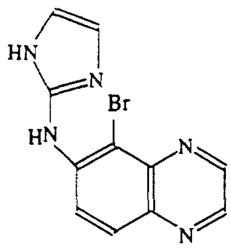 | 100,000 | 512 | 12,910 | 9,673 | 49.4 |
| 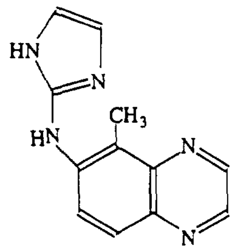 | 30,550 | 31 | 1,156 | 212 | 35.3 |
| 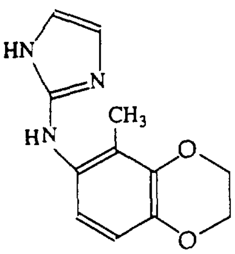 | 2,147 | 1.7 | 82 | 19 | 1.61 |
| 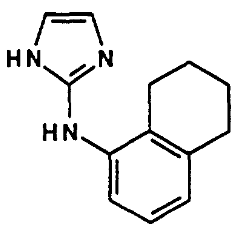 | 4,980 | 39 | 152 | 252 | |
| 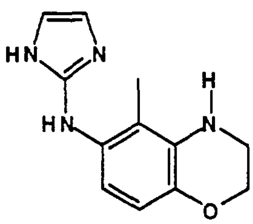 | 14,784 | 6.3 | 436 | 229 | |
| 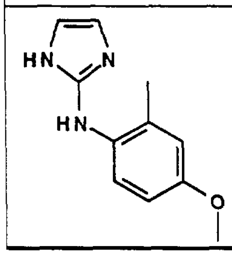 | 7,613 | 10 | 434 | 512 | |

Several modifications of the above described compounds, the processes disclosed for making them, and application of the disclosed processes to numerous compounds beyond the examples set forth above, may be practiced by those skilled in the art without departing from the scope and spirit of the present invention. Therefore the scope of the present invention should be interpreted solely from the following claims, as such claims are read in light of the present disclosure.

**Claims**

1. The compound of formula I

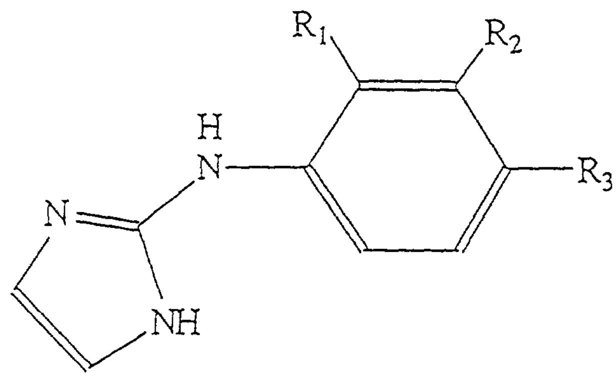

formula I

wherein $R_1$ is alkyl or alkenyl of 1 to 4 carbons, cyclopropyl, $-OR_4$, $-SR_4$, $-NR_4R_4$ or $-Si(R_4)_3$ wherein $R_4$ represents alkyl or alkenyl of 1 to 4 carbon atoms or is hydrogen, $R_2$ and $R_3$ are selected from the group consisting of $R_1$ and hydrogen; or $R_1$ and $R_2$ taken together form a fused ring with the phenyl moiety and are selected from the group consisting of $-(CH_2)_4-$, $-NH(CH_2)_2NH-$, $-O(CH_2)_2O-$,$-NH(CH_2)_2O-$, $-S(CH_2)_2S-$, $-NH(CH_2)S-$, and $-O(CH_2)_2S-$ and $R_3$ is selected from the group consisting of $R_1$ and hydrogen; or $R_2$ and $R_3$ taken together form a fused ring with the phenyl moiety and are selected from the group consisting of-$(CH_2)_4-$, $-NH(CH_2)_2NH-$, $-O(CH_2)_2O-$, $-NH(CH_2)_2O-$, $-S(CH_2)_2S-$, $-NH(CH_2)S-$, and-$O(CH_2)_2S-$, -CH=CH-CH=CH-, -N=CH-CH=N- and -N=CH-N=CH or pharmaceutically acceptable salts thereof.

2. The compound of claim 1 wherein the phenyl ring is monocyclic and the Hammett $\sigma_{meta}$ value for each substituent at $R_1$, $R_2$ and $R_3$ is 0.4 or less, and $R_2$ and $R_3$ are optionally hydrogen.

3. The compound of claim 2 wherein the Hammett $\sigma_{meta}$ value for the substituent described by $R_1$ is 0.15 or less.

4. The compound of claim 1 wherein $R_1$ is selected from the group consisting of methyl, ethyl, n-propyl, isopropyl, hydroxy, methoxy, ethoxy, thiol, methanethiol, ethanethiol, amino, N-methylamino, N-N-dimethylamino and N-ethylamino and N-N-diethylamino.

5. The compound of claim 1 wherein the $R_2$ and $R_3$ groups together represent a ring fused to the phenyl ring and $R_1$ has a Hammett $\sigma_{meta}$ value of 0.4 or less.

6. The compound of claim 5 wherein $R_1$ has a Hammett $\sigma_{meta}$ value of 0.15 or less.

7. The compound of claim 1 wherein $R_1$ and $R_2$ together represent $-CH_2-CH_2-CH_2-CH_2-$.

8. The compound of claim 1 wherein $R_2$ and $R_3$ taken together are selected from the group consisting of $-NH-CH_2-CH_2-O-$, $-O-CH_2-CH_2-O-$, -N=CH-CH=N- and-$NH-CH_2-CH_2-NH-$, and $R_1$ has a $\sigma_{meta}$ value of 0.15 or less.

9. The compound of claim 1 which is 1-methoxy-3-methyl-4-(2-imidazolyl)amino-benzene.

10. The compound of claim 1 which is selected from the group consisting of 5-methyl-6-N-(2-imidazolyl)amino-1,4-benzodioxane; 5-methyl-6-N-(2-imidazolyl)amino-2,3-dihydro-1,4-benzoxazine; and 5-methyl-6-N-(2-imidazolyl)amino-quinoxaline; and 5-methyl-6-N-(2-imidazolyl)amino-2,3-dihydro-quinoxaline.

**11.** A compound for use in selectively stimulating $\alpha_{2A}$ adrenoceptors in a mammal in need of such stimulation to attain a therapeutic effect the compound being a compound of formula I

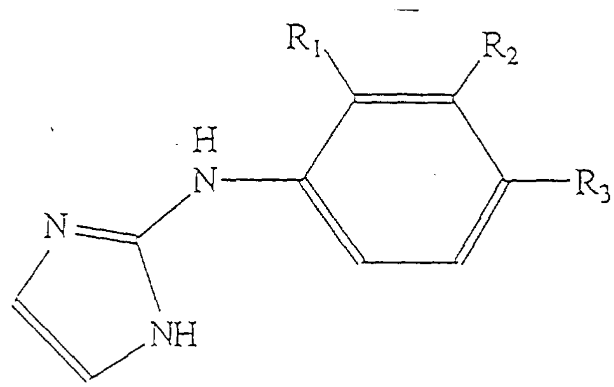

formula I

wherein $R_1$ is alkyl or alkenyl of 1 to 4 carbons, cyclopropyl, $-OR_4$, $-SR_4$, $-NR_4R_4$ or $-Si(R_4)_3$ wherein $R_4$ represents alkyl or alkenyl of 1 to 4 carbon atoms or is hydrogen, $R_2$ and $R_3$ are selected from the group consisting of $R_1$ and hydrogen; or $R_1$ and $R_2$ taken together form a fused ring with the phenyl moiety and are selected from the group consisting of $-(CH_2)_4-$, $-NH(CH_2)_2NH-$, $-O(CH_2)_2O-$,$-NH(CH_2)_2O-$, $-S(CH_2)_2S-$, $-NH(CH_2)S-$, and $-O(CH_2)_2S-$ and $R_3$ is selected from the group consisting of $R_1$ and hydrogen; or $R_2$ and $R_3$ taken together form a fused ring with the phenyl moiety and are selected from the group consisting of-$(CH_2)_4-$, $-NH(CH_2)_2NH-$, $-O(CH_2)_2O-$, $-NH(CH_2)_2O-$, $-S(CH_2)_2S-$, $-NH(CH_2)S-$, and-$O(CH_2)_2S-$, -CH=CH-CH=CH-, -N=CH-CH=N- and -N=CH-N=CH-, or a mixture of compounds thereof, or pharmaceutically acceptable salts thereof.

**12.** The compound for use according to claim 11 wherein the phenyl ring is monocyclic and the Hammett $\sigma_{meta}$ value for each substituent at $R_1$, $R_2$ and $R_3$ is 0.15 or less with $R_2$ or $R_3$ optionally being hydrogen.

**13.** The compound for use according to claim 11 wherein $R_1$ is selected from the group consisting of methyl, ethyl, n-propyl, isopropyl, hydroxy, methoxy, ethoxy, thiol, methanethiol, ethanethiol, amino, N-methylamino, N-N-dimethylamino and N-ethylamino and N-N-diethylamino.

**14.** The compound for use according to claim 11 wherein the therapeutic effect is chosen from the group consisting of: reduction or maintenance of intraocular pressure in at least one eye; treatment of peripheral pain; augmentation of the effects of anesthetics; treatment of hypertension; treatment of hyperglycemia; and sedation.

**15.** The compound for use according to claim 11 wherein the $R_2$ and $R_3$ groups together represent a ring fused to the phenyl ring and $R_1$ has a Hammett $\sigma_{meta}$ value of 0.15 or less.

**16.** The compound for use according to claim 11 wherein $R_1$ and $R_2$ together represent $-CH_2-CH_2-CH_2-CH_2-$.

**17.** The compound for use according to claim 11 wherein $R_2$ and $R_3$ taken together are selected from the group consisting of $-NH-CH_2-CH_2-O-$, $-O-CH_2-CH_2-O-$, -N=CH-CH=N- and-$NH-CH_2-CH_2-NH-$, and $R_1$ has a $\sigma_{meta}$ value of 0.15 or less.

**18.** The compound for use according to claim 11 wherein formula I represents 1-methoxy-3-methyl-4-(2-imidazolyl) amino benzene.

**19.** The compound for use according to claim 11 wherein formula I is selected from the group consisting of 5-methyl-6-N-(2-imidazolyl)amino-1,4-benzodioxane; and 5-methyl-6-N-(2-imidazolyl)amino-2,3-dihydro-1,4-benzoxazine; and 5-methyl-6-N-(2-imidazolyl)amino-quinoxaline; and 5-methyl-6-N-(2-imidazolyl)amino-2,3-dihydroquinoxaline.

20. The use of a compound as defined in any one of claims 1 to 10 for the manufacture of a medicament for use in mammals for the maintenance or reduction of intraocular pressure; for the treatment of peripheral pain; for the augmentation of the effects of anesthetics; for the treatment of hypertension; for the treatment of hyperglycemia; and as a sedative.

**Patentansprüche**

1. Verbindung der Formel I

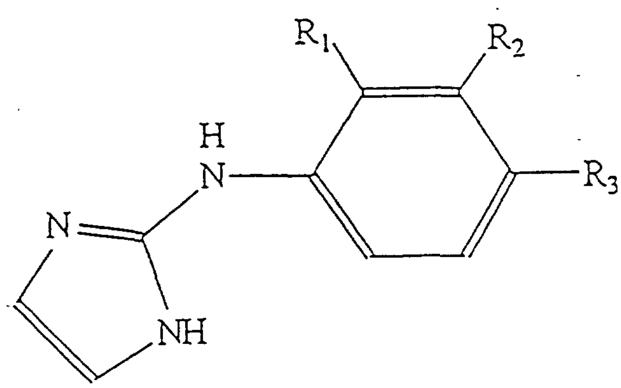

Formel I

wobei $R_1$ Alkyl oder Alkenyl von 1 bis 4 Kohlenstoffatomen, Cyclopropyl, $-OR_4$, $-SR_4$, $-NR_4R_4$ oder $-Si(R_4)_3$ ist, wobei $R_4$ Alkyl oder Alkenyl von 1 bis 4 Kohlenstoffatomen darstellt oder Wasserstoff ist, wobei $R_2$ und $R_3$ aus der Gruppe ausgewählt sind, die aus $R_1$ und Waserstoff besteht;
oder wobei $R_1$ und $R_2$ zusammengenommen einen kondensierten Ring mit dem Phenylanteil bilden und aus der Gruppe ausgewählt sind, die aus $-(CH_2)_4-$, $-NH(CH_2)_2NH-$, $-O(CH_2)_2O-$, $-NH(CH_2)_2O-$, $-S(CH_2)_2S-$, $-NH(CH_2)-$
und $-O(CH_2)_2S-$ besteht und $R_3$ aus der Gruppe ausgewählt ist, die aus $R_1$ und Wasserstoff besteht; oder $R_2$ und $R_3$ zusammengenommen einen kondensierten Ring mit dem Phenylanteil bilden und aus der Gruppe ausgewählt sind, die aus $-(CH_2)_4-$, $-NH(CH_2)_2NH-$, $-O(CH_2)_2O-$, $-NH(CH_2)_2O-$,$-S(CH_2)_2S-$, $-NH(CH_2)S-$ und $-O(CH_2)_2S-$,-CH=CH-CH=CH-,-N=CH-CH=N- und -N=CH-N=CH oder pharmazeutisch akzeptablen Salzen hiervon, bestehen.

2. Verbindung nach Anspruch 1,
bei der der Phenylring monozyklisch ist und der Hammett $\sigma_{meta}$-Wert für jeden Substituenten an $R_1$, $R_2$ und $R_3$ 0,4 oder weniger ist und $R_2$ und $R_3$ wahlweise Wasserstoff sind.

3. Verbindung nach Anspruch 2,
bei der der Hammett $\sigma_{meta}$-Wert für den Substituenten, der durch $R_1$ beschrieben ist, 0,15 oder weniger beträgt.

4. Verbindung nach Anspruch 1,
bei der $R_1$ aus der Gruppe ausgewählt ist, die aus Methyl, Ethyl, n-Propyl, Isopropyl, Hydroxy, Methoxy, Ethoxy, Thiol, Methanthiol, Ethanthiol, Amino, N-Methylamino, N,N-Dimethyl-amino und N-Ethylamino und N-N-Diethylamino besteht.

5. Verbindung nach Anspruch 1,
bei der $R_2$ und $R_3$-Gruppen zusammen einen Ring darstellen, der an den Phenylring kondensiert ist und $R_1$ einen Hammett $\sigma_{meta}$-Wert von 0,4 oder weniger aufweist.

6. Verbindung nach Anspruch 5,
bei der $R_1$ einen Hammett $\sigma_{meta}$-Wert von 0,15 oder weniger aufweist.

**7.** Verbindung nach Anspruch 1,
bei der $R_1$ und $R_2$ zusammen $-CH_2-CH_2-CH_2-CH_2-$darstellen.

**8.** Verbindung nach Anspruch 1,
bei der $R_2$ und $R_3$ zusammengenommen aus der Gruppe ausgewählt sind, die aus $-NH-CH_2-CH_2-O-$, $-O-CH_2-CH_2-O-$, $-N=CH-CH=N-$ und $-NH-CH_2-CH_2-NH-$ besteht, und $R_1$ einen $\sigma_{meta}$-Wert von 0,15 oder weniger aufweist.

**9.** Verbindung nach Anspruch 1,
die 1-Methoxy-3-methyl-4-(2-imidazolyl)aminobenzol ist.

**10.** Verbindung nach Anspruch 1,
die aus der Gruppe ausgewählt ist, die aus 5-Methyl-6-N-(2-imidazolyl)amino-1,4-benzodioxan, 5-Methyl-6-N-(2-imidazolyl) amino-2,3-dihydro-1,4-benzoxazin und 5-Methyl-6-N-(2-imidazolyl)aminochinoxalin und 5-Methyl-6-N-(2-imidazolyl)amino-2,3-dihydrochinoxalin besteht.

**11.** Verbindung zur Verwendung in der selektiven Stimulierung von $\alpha_{2A}$-Adrenorezeptoren bei einem Säugetier, das einer derartigen Stimulierung bedarf, um eine therapeutische Wirkung zu erzielen, wobei die Verbindung eine Verbindung der Formel I ist

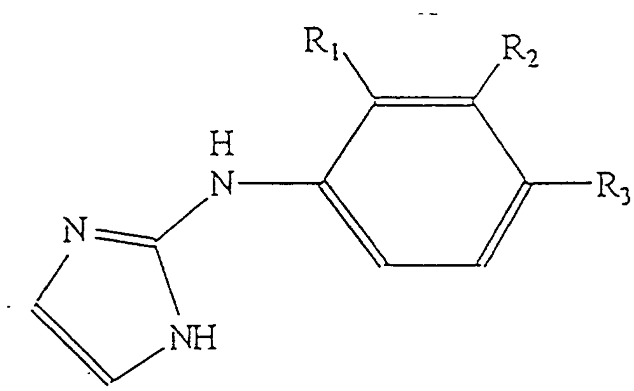

Formel I

wobei $R_1$ Alkyl oder Alkenyl von 1 bis 4 Kohlenstoffatomen, Cyclopropyl, $-OR_4$, $-SR_4$, $-NR_4R_4$ oder $-Si(R_4)_3$ ist,

wobei $R_4$ Alkyl oder Alkenyl von 1 bis 4 Kohlenstoffatomen darstellt oder Wasserstoff ist, wobei $R_2$ und $R_3$ aus der Gruppe ausgewählt sind, die aus $R_1$ und Waserstoff besteht;

oder wobei $R_1$ und $R_2$ zusammengenommen einen kondensierten Ring mit dem Phenylanteil bilden und aus der Gruppe ausgewählt sind, die aus $-(CH_2)_4-$, $-NH(CH_2)_2NH-$, $-O(CH_2)_2O-$, $-NH(CH_2)_2O-$, $-S(CH_2)_2S-$, $-NH(CH_2)S-$

und $-O(CH_2)_2S-$ besteht, und wobei $R_3$ aus der Gruppe ausgewählt ist, die aus $R_1$ und Wasserstoff besteht; oder wobei $R_2$ und $R_3$ zusammengenommen einen kondensierten Ring mit dem Phenylanteil bilden und aus der Gruppe ausgewählt sind, die aus $-(CH_2)_4-$, $-NH(CH_2)_2NH-$, $-O(CH_2)_2O-$, $-NH(CH_2)_2O-$, $-S(CH_2)_2S-$, $-NH(CH_2)S-$ und $-O(CH_2)_2S-$, $-CH=CH-CH=CH-$, $-N=CH-CH=N-$ und $-N=CH-N=CH$ besteht, oder einem Gemisch von Verbindungen hiervon, oder pharmazeutisch akzeptablen Salzen hiervon.

**12.** Verbindung zur Verwendung nach Anspruch 11,
bei der der Phenylring monozyklisch ist und der Hammett $\sigma_{meta}$-Wert für jeden Substituenten an $R_1$, $R_2$ und $R_3$ 0,15 oder weniger ist, wobei $R_2$ oder $R_3$ optional Wasserstoff ist.

**13.** Verbindung zur Verwendung nach Anspruch 11,
wobei $R_1$ aus der Gruppe ausgewählt ist, die aus Methyl, Ethyl, n-Propyl, Isopropyl, Hydroxy, Methoxy, Ethoxy, Thiol, Methanthiol, Ethanthiol, Amino, N-Methylamino, N,N-Dimethyl-amino und N-Ethylamino und N-N-Diethylamino besteht.

**14.** Verbindung zur Verwendung nach Anspruch 11, bei der die therapeutische Wirkung aus der Gruppe ausgewählt ist, die aus: Reduzierung oder Aufrechterhaltung des Augeninnendrucks in zumindest einem Auge, Behandlung von peripherem Schmerz, Vergrößerung der Wirkungen von Anästhetika, Behandlung des Hochdrucks, Behandlung der Hyperglykämie und der Sedierung besteht.

**15.** Verbindung zur Verwendung nach Anspruch 11, bei der $R_2$- und $R_3$-Gruppen zusammen einen Ring darstellen, der an den Phenylring kondensiert ist und bei der $R_1$ einen Hammett $\sigma_{meta}$-Wert von 0,15 oder weniger aufweist.

**16.** Verbindung zur Verwendung nach Anspruch 11, bei der $R_1$ und $R_2$ zusammen $-CH_2-CH_2-CH_2-CH_2-$darstellen.

**17.** Verbindung zur Verwendung nach Anspruch 11, bei der $R_2$ und $R_3$ zusammengenommen aus der Gruppe ausgewählt sind, die aus $-NH-CH_2-CH_2-O-$, $-O-CH_2-CH_2-O-$, -N=CH-CH=N- und $-NH-CH_2-CH_2-NH-$ besteht, und bei der $R_1$ einen $\sigma_{meta}$-Wert von 0,15 oder weniger aufweist.

**18.** Verbindung zur Verwendung nach Anspruch 11, bei der Formel I 1-Methoxy-3-methyl-4-(2-imidazolyl)amino-benzol darstellt.

**19.** Verbindung zur Verwendung nach Anspruch 11, bei der Formel I aus der Gruppe ausgewählt ist, die aus 5-Methyl-6-N-(2-imidazolyl)amino-1,4-benzodioxan, 5-Methyl-6-N-(2-imidazolyl)amino-2,3-dihydro-1,4-benzoxazin und 5-Methyl-6-N-(2-imidazolyl)aminochinoxalin und 5-Methyl-6-N-(2-imidazolyl)amino-2,3-dihydrochinoxalin besteht.

**20.** Verwendung einer Verbindung, wie sie in einem der Ansprüche 1 bis 10 definiert ist, zur Herstellung eines Medikaments zur Verwendung bei Säugetieren, zur Aufrechterhaltung oder Reduzierung des Augeninnendrucks; zur Behandlung von periphärem Schmerz; zur Steigerung der Wirkungen von Anästhetika, zur Behandlung des Hochdrucks, zur Behandlung der Hyperglykämie und als Sedativum.

## Revendications

**1.** Composé de la formule I

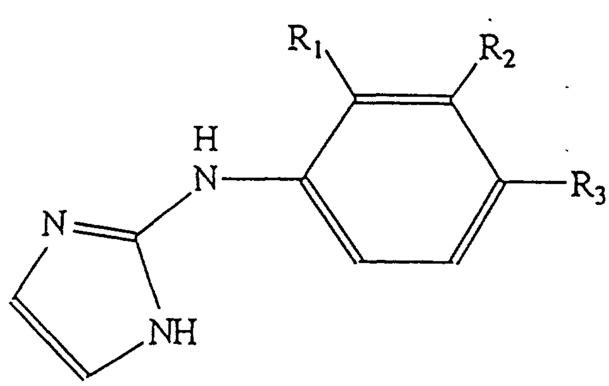

formula I

où $R_1$ est un alkyle ou alkényle de 1 à 4 atomes de carbone, cyclopropyle, $-OR_4$, $-SR_4$, $-NR_4R_4$ ou $-Si(R_4)_3$ où $R_4$ représente un alkyle ou alkényle de 1 à 4 atomes de carbone ou est de l'hydrogène, $R_2$ et $R_3$ sont choisis dans le groupe consistant en R1 et de l'hydrogène; ou $R_1$ et $R_2$ pris ensemble forment un anneau condensé avec le groupe fonctionnel phényle et sont choisis dans le groupe consistant en $-(CH_2)_4-$, $-NH\ (CH_2)_2NH-$, $-O(CH_2)_2O-$, $-NH(CH_2)_2O-$, $-S(CH_2)_2S-$, $-NH(CH_2)S-$, et $-O(CH_2)_2S-$ et $R_3$ est choisi dans le groupe consistant en $R_1$ et hydrogène; ou $R_2$ et $R_3$ pris ensemble forment un anneau condensé avec le groupement fonctionnel phényle et sont

choisis dans le groupe consistant en $-(CH_2)_4-$, -NH $(CH_2)_2NH-$, $-O(CH_2)_2O-$, $-NH(CH_2)_2O-$, $-S(CH_2)_2S-$, $-NH(CH_2)$ S-, et $-O(CH_2)_2S-$, -CH=CH-CH=CH-,-N=CH-CH=N et -N=CH-N=CH ou des sels pharmaceutiquement acceptables de ceux-ci.

**2.** Composé de la revendication 1 où l'anneau phényle est monocyclique et l'indice de Hammett $\sigma_{meta}$ de chaque substituant à $R_1$, $R_2$ et $R_3$ est 0,4 ou moins, et $R_2$ et $R_3$ sont facultativement de l'hydrogène.

**3.** Composé selon la revendication 2 où l'indice de Hammett $\sigma_{meta}$ pour le substituant décrit par $R_1$ est 0,15 ou moins.

**4.** Composé selon la revendication 1 où $R_1$ est choisi dans le groupe consistant en méthyle, éthyle, n-propyle, isopropyle, hydroxy, méthoxy, éthoxy, thiol, méthanethiol, éthanethiol, amino, N-méthylamino, N-N-diméthylamino, N-éthylamino et N-N-diéthylamino.

**5.** Composé selon la revendication 1 où les groupes $R_2$ et $R_3$ ensemble représentent un anneau condensé à l'anneau phényle et $R_1$ a un indice de Hammett $\sigma_{meta}$ de 0,4 ou moins.

**6.** Composé selon la revendication 5 où $R_1$ a un indice de Hammett $\sigma_{meta}$ de 0,15 ou moins.

**7.** Composé selon la revendication 1 où $R_1$ et $R_2$ ensemble représentent $-CH_2-CH_2-CH_2-CH_2$.

**8.** Composé selon la revendication 1 où $R_2$ et $R_3$ pris ensemble sont choisis dans le groupe consistant en $-NH-CH_2-CH_2-O-$, $-O-CH_2-CH_2-O-$, -N=CH-CH=N- et $NH-CH_2-CH_2-NH-$, et $R_1$ a un indice $\sigma_{meta}$ de 0,15 ou moins.

**9.** Composé selon la revendication 1 qui est le 1-méthoxy-3-méthyl-4-(2-imidazolyl)amino-benzène.

**10.** Composé selon la revendication 1 qui est choisi dans le groupe consistant en 5-méthyl-6-N-(2-imidazolyl) amino-1,4-benzodioxane; 5-méthyl-6-N-(2-imidazolyl)amino-2, 3-dihydro-1, 4-benzoxazine; et 5-méthyl-6-N-(2-imidazolyl) amino-quinoxaline; et 5-méthyl-6-N-(2-imidazolyl)amino-2,3-dihydro-quinoxaline.

**11.** Un composé à utiliser pour stimuler sélectivement les $\alpha_{2A}$ adrénocepteurs chez les mammifères en nécessité d'une telle stimulation pour atteindre un effet thérapeutique, le composé étant un composé de la formule I

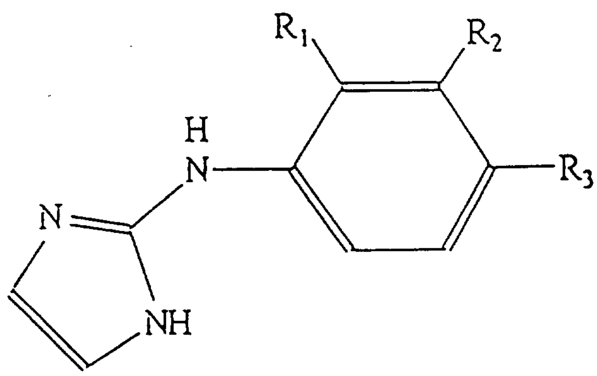

formula I

où $R_1$ est un alkyle ou alkényle de 1 à 4 atomes de carbone, cyclopropyle, $-OR_4$, $-SR_4$, $-NR_4R_4$ ou $-Si(R_4)_3$ où- $R_4$ représente un alkyle ou alkényle de 1 à 4 atomes de carbone ou est de l'hydrogène, $R_2$ et $R_3$ sont choisis dans le groupe consistant en R1 et de l'hydrogène; ou $R_1$ et $R_2$ pris ensemble forment un anneau condensé avec le groupe fonctionnel phényle et sont choisis dans le groupe consistant en $-(CH_2)_4-$, -NH $(CH_2)_2NH-$, $-O(CH_2)_2O-$, $-NH(CH_2)_2O-$, $-S(CH_2)_2S-$, $-NH(CH_2)S-$, et $-O(CH_2)_2S-$ et $R_3$ est choisi dans le groupe consistant en $R_1$ et hydrogène; ou $R_2$ et $R_3$ pris ensemble forment un anneau condensé avec le groupement fonctionnel phényle et sont choisis dans le groupe consistant en $-(CH_2)_4-$, -NH $(CH_2)_2NH-$, $-O(CH_2)_2O-$, -NH $(CH_2)_2O-$, $-S(CH_2)_2S-$, $-NH(CH_2)$ S-, et $-O(CH_2)_2S-$, -CH=CH-CH=CH-, -N=CH-CH=N- et -N=CH-N=CH- ou un mélange de composés de ceux-ci ou des sels pharmaceutiquement acceptables de ceux-ci.

**12.** Composé à utiliser selon la revendication 11 où l'anneau phényle est monocyclique et l'indice de Hammett $\sigma_{meta}$ pour chaque substituant en $R_1$, $R_2$ et $R_3$ est 0,15 ou moins, et $R_2$ et $R_3$ sont facultativement de l'hydrogène.

**13.** Composé à utiliser selon la revendication 11 où $R_1$ est choisi dans le groupe consistant en méthyle, éthyle, n-propyle, isopropyle, hydroxy, méthoxy, éthoxy, thiol, méthanethiol, éthanethiol, amino, N-méthylamino, N-N-diméthylamino et N-éthylamino et N-N-diéthylamino.

**14.** Composé à utiliser selon la revendication 11 où l'effet thérapeutique est choisi dans le groupe consistant en une diminution ou maintien de la pression intra-occulaire dans au moins un oeil; traitement de la douleur périphérique; augmentation des effets des anesthésiques; traitement de l'hypertension; traitement de l'hyperglycémie; et sédation.

**15.** Composé à utiliser selon la revendication 11 où les groupes $R_2$ et $R_3$ ensemble représentent un anneau condensé à l'anneau phényle et $R_1$ a un indice de Hammett $\sigma_{meta}$ de 0,15 ou moins.

**16.** Composé à utiliser selon la revendication 11 où $R_1$ et $R_2$ ensemble représentent $-CH_2-CH_2-CH_2-CH_2-$.

**17.** Composé à utiliser selon la revendication 11 où $R_2$ et $R_3$ pris ensemble sont choisis dans le groupe consistant en $-NH-CH_2-CH_2-O-$, $-O-CH_2-CH_2-O-$, -N=CH-CH=N- et $NH-CH_2-CH_2-NH-$, et $R_1$ a un indice $\sigma_{meta}$ de 0,15 ou moins.

**18.** Composé à utiliser selon la revendication 11 où la formule I représente le 1-méthoxy-3-méthyl-4-(2-imidazolyl) amino-benzène.

**19.** Composé à utiliser selon la revendication 11 où la formule I est choisie dans le groupe consistant en 5-méthyl-6-N-(2-imidazolyl) amino-1,4-benzodioxane; 5-méthyl-6-N-(2-imidazolyl)amino-2, 3-dihydro-1, 4-benzoxazine; et 5-méthyl-6-N-(2-imidazolyl) amino-quinoxaline; et 5-méthyl-6-N-(2-imidazolyl)amino-2,3-dihydro-quinoxaline.

**20.** Utilisation d'un composé tel que défini dans l'une quelconque des revendications 1 à 10 pour la fabrication d'un médicament utilisé chez les mammifères pour le maintien ou la diminution de la pression intra-occulaire, pour le traitement de la douleur périphérique; pour l'augmentation des effets des anesthésiques; pour le traitement de l'hypertension; pour le traitement de l'hyperglycémie; et comme sédatif.